# EUROPEAN PATENT SPECIFICATION

(11) **EP 4 173 600 B1**
(45) Date of publication and mention of the grant of the patent: **17.06.2026**
(21) Application number: 20891458.0
(22) Date of filing: 07.12.2020
(51) Int. Cl.: A61F 2/95

(54) **BODY OF A DEVICE FOR POSITIONING A CORONARY STENT IN CORONARY ARTERIES AND PULLING A TRANSDUCER OF AN INTRAVASCULAR ULTRASOUND CATHETER**
KÖRPER EINER VORRICHTUNG ZUM POSITIONIEREN EINES KORONARSTENTS IN KORONARARTERIEN UND ZUM ZIEHEN EINES WANDLERS EINES INTRAVASKULÄREN ULTRASCHALLKATHETERS
CORPS DE DISPOSITIF POUR LE POSITIONNEMENT D'UN STENT CORONAIRE DANS DES ARTÈRES CORONAIRES ET DE DÉPLOIEMENT D'UN CAPTEUR DE CATHÉTER POUR RECHERCHE ULTRASONORE INTRAVASCULAIRE

(30) Priority: 30.06.2020 RU 2020121738
(43) Date of publication of application: 03.05.2023
(73) Proprietor: Seven Sons Ltd., Tel Aviv 36744 (IL)
(72) Inventor: TRAPEZNIKOV, Vladimir Borisovich, Moscow, 121165 (RU)
(74) Representative: Jeck, Jonathan
(86) International application number: PCT/RU2020/000666
(87) International publication number: WO 2022/005323

(56) References cited:
- RU-C1- 2 650 038
- RU-C1- 2 652 732
- RU-C1- 2 710 216
- US-A1- 2007 191 865
- US-A1- 2014 128 963
- US-A1- 2019 328 560
- US-A1- 2020 146 857

## Description

This invention relates to a body of the device suitable for use in medicine to position a coronary stent in the most accurate, fast and safe manner on a delivery system to carry out endovascular coronary stenting or pull a catheter transducer for intravascular ultrasound (IVUS).

When the device is used to pull the catheter transducer, such catheter transducer is installed in the device instead of the coronary stent delivery system in much the same manner.

For intravascular imaging, the guiding catheter is suitably installed within the coronary ostium so that the guiding catheter can be pulled through the target narrowing to distal arteries. The device is placed on the digital IVU Scatheter in the same manner as it normally placed on the coronary stent delivery system. A mechanical transducer of the Revolution (Volcano (Philips)) type or other similar devices cannot be currently used due to specific features of their pulling mechanisms. The device is positioned as close as possible to the proximal section of the transmitting catheter, i.e., the section where the transmitting catheter is connected by means of a wire to a station or built-in interface. The transducer is moved through the guide to a distance 30 - 40 mm more distal than the target segment. When in the appropriate zone, the device is fixed on the digital IVUS catheter whereupon the device body is rotated to slowly move the device using the procedure similar to the stent positioning procedure for high-precision estimation of this section.

Known on the medical equipment market are devices proposed by the same applicant to position stents in the coronary arteries (RU2598798, RU2652732, RU2650038, RU2661092, RU2710216),with the bodies of such devices differing from the proposed invention the advantages of which are as follows:
- easy and short-time assembly of the device body owing to a brace and a ring and because there is no need for glue to mate the parts;
- higher strength of the product owing to the brace and a large number of tapered projections and their mating tapered holes in the front portion of the device body;
- greater rigidity of the front portion of the device body when a retainer operates, such rigidity provided by a thicker wall of the front truncated portion of the body in the retainer lug zone, with the maximum possible number of the tapered projections and mating tapered holes made along the edges of the thick wall;

- no need for a tube to position a coronary stent on the delivery system inside the device, such positioning provided by a fillet inside the front truncated portion of the body and chamfers on the rear end portion of the body and a runner in their central hole;
- improved performance of the product during coronary stent positioning owing to greater rigidity of the runner guides provided by an additional wall interconnecting the guides;
- no need for gluing and enhanced mating force of the guides for the runner of the front portion of the body owing to transverse walls provided at the end of the guides, with a tapered projection and mating tapered hole;
- possibility of use of the device body to pull the catheter transducer;
- greater rigidity of coupling of the halves of the rear portion of the body owing to additional longitudinal slots and longitudinal projections along the edge of the internal side of the rear portion of the body in the overlap zone;
- maintenance of integrity of the device body in the course of operation owing to the brace securely retained by the projections at the end of the rear portion of the device body;
- when the coronary stent is being positioned or catheter transducer is being pulled, the rear portion of the device body can be readily rotated with the aid of a ring owing to parts engagement;
- when the coronary stent is being positioned, the brace cannot be displaced around the rear portion of the body owing to the longitudinal projections on the external side of the rear portion of the body in the internal thread zone and internal guides on the brace;
- no need for accurate positioning of the brace with respect to the rear portion of the body when the body of the device is being assembled; simple and fast assembly owing to specially shaped longitudinal projections on the external side of the rear portion of the device body in the internal thread zone;
- reduced load to the operator's fingers to close the retainer owing to a wall directed at an angle to a point wall projection provided in the opening for the retainer lugs and designed to hold the retainer lugs in the extreme position.

The claimed invention is industrially applicable.

The subject matter of the present invention is a body of the device to position a coronary stent on a delivery system in coronary arteries or pull a catheter transducer for intravascular ultrasound.

The body is composed of a retainer with a thickening with lugs, a runner with a hole to accommodate a delivery system with projections designed to engage an internal double-start thread of a rear cylindrical portion of the body and with projections used for movement along guides inside the body on one side, and a cap with a hole to accommodate a delivery system with an elastic bushing with a hole to position the delivery system inside with a cone-shaped and end surfaces corresponding to similar surfaces inside the cap and at the end of the runner, installed in the threaded cap to be screwed on the runner on the other side.

A mechanism housed in the body is a subject matter of an independent patent (Patent RU 2661092) of the same applicant and cited herein solely for illustration and better understanding of the claimed invention.

The device body is composed of two components one of which is essentially a part comprising a front portion truncated on one side thereof and a cylindrical portion on the opposite side thereof, a smooth cylindrical portion of a smaller diameter and guides for a runner, said component forming the front portion of the body, and the other component includes a part arranged over the smooth cylindrical portion of the first component and a rear part with the internal double-start thread and a chamfered end face, said component forming the rear portion of the body.

The front portion of the body is truncated on the front side thereof and continuously extends on the opposite side thereof in the form of a cylindrical portion, smooth cylindrical portion of a smaller diameter and runner guides interconnected by longitudinal walls and provided at the end with transversal walls having tapered holes and mating tapered projections to couple the body halves.

The front truncated portion of the body is adapted to be held by the fingers of the surgeon's left hand and provided to this end with recesses allowing the surgeon to fix the device in the desired position, with the recesses also serving as a pad to fix a coronary catheter. For more convenient arrangement of the coronary stent delivery system and catheter transducer, the walls in the front truncated portion of the body are made rounded on the internal side opposite to the recesses for the operator's left hand fingers where said walls contact the mouth of the hole in which the delivery system is installed.

Square openings for retainer lugs are provided on both sides of the front cylindrical portion of the body and the walls of the front portion of the body are made thicker within the section between the openings for the retainer lugs. In each square opening two point projections are located on one side at a distance equal to the retainer lug width and designed to hold the retainer lugs in the extreme position and the retainer proper in the closed position, each projection having one flat wall which is directed at an angle to the projection on the side of the retainer open position and along which the retainer lug slides when it changes its position, whereby the force to be applied by the operator's hand to rotate the retainer lugs to close the mechanism is decreased.

The front smooth cylindrical portion of the body has a ring suitably mounted thereon.

The ring has a recess made along the entire internal circumference thereof and designed to receive mating projections located along the circumference of the halves of the rear portion of the body on the side of the retainer so as couple these halves.

On the external circumference of the ring, trapezoidal slots are provided to receive the mating projections of the front end of the rear portion of the body, said projections having a trapezoidal form and designed to fix the rear portion of the body in the ring to prevent its spontaneous rotation while allowing the operator to rotate the rear portion of the body relative to the front portion of the body by the ring.

A cylindrical slot with point elements is made along the circumference of the end face of the smooth cylindrical portion of the front portion of the body on the side of the runner guide, said cylindrical slot designed for coupling with the mating projection with point elements made along the circumference of the internal smooth cylindrical surface of the rear portion of the body on the side of the surface with the double-start thread.

The rear cylindrical portion of the body has longitudinal projections and mating longitudinal slots located on the internal side thereof along the edges of the halves in the zone of the smooth cylindrical surface and used to couple the halves of the rear portion of the body.

Point elements arranged on the internal smooth cylindrical surface of the rear portion of the body along the circumference on the retainer side are designed to reduce friction between the rotatable portions of the body.

A cylindrical projection with point elements is provided along the circumference between the smooth internal surface and the surface with the internal double-start thread of the rear portion of the body to engage the cylindrical slot of the smooth cylindrical part of the front portion of the body on the runner guide side so that the rear cylindrical portion of the body can be rotated about the axis thereof.

Longitudinal projections are made on the external surface of the rear portion of the body, with the projection ends smoothly curved in the opposite directions in order to center the brace fitted over the rear portion of the body and to prevent spontaneous rotation of the brace. Projections to fix the brace in the brace recesses along the circumference of the brace end face are located on the rear end face of the body.

The brace is fitted over the rear portion of the body in the zone of the internal double-start thread.

The brace is essentially a hollow cylinder with internal longitudinal guides along the whole length to ensure snug fit to the external surface of the rear portion of the body on the external side of which mating longitudinal projections are provided having the ends thereof smoothly curved in the opposite directions and used to securely attach the brace.

Recesses made along the circumference of the brace end face serve to fix the brace on the surface of the rear portion of the body with the aid of mating projections along the circumference of the end face of the rear portion of the body.

Through holes are made in the front portion of the body, runner, cap, elastic bushing and end face of the rear portion of the body to introduce the coronary stent delivery system or catheter transducer into the device installed in the body.

For easy positioning of the coronary stent on the delivery system or catheter transducer inside the device in the central through hole, chamfers are made on the side of the runner end face and in the rear portion of the body.

The body is made of pressure cast plastic and comprises the front and rear portions, each portion composed of two halves.

Each half of the front and rear portions of the body is solid cast and the two halves are coupled after assembly of the mechanism with the aid of the tapered projections and mating tapered holes made along the internal edges of the front and rear portions of the body and on the transverse walls at the end of the runner guides. The maximum possible number of the tapered projections and mating tapered holes is made on the tapered protrusion of the front portion of the body and in the zone between the openings for the retainer lugs.

The halves of the front portion of the body are coupled with the aid of the tapered projections and mating tapered holes made along the internal edge of the whole body and the tapered projections and mating tapered holes made in the transverse walls at the end of the guides; after the halves of the front portion of the body are coupled, a ring is fitted over the smooth cylindrical portion of the front portion of the body on the side of the guides for the runner, said ring having a recess in which the mating projections of the semicircles of the halves of the rear portion of the body are inserted whereas in the trapezoidal slots made on the external circumference of the ring the mating trapezoidal projections on the semicircles of the front portion of the body are inserted whereupon the halves are coupled so that the smooth cylindrical portion of the front portion of the body is accommodated in the halves of the rear smooth cylindrical portion of the body, with the cylindrical projection with point elements located between the internal smooth portion of the rear portion of the body and the portion having the internal double-start thread being inserted in the cylindrical slot of the smooth cylindrical portion of the front portion of the body on the side of the runner guides and the halves being coupled in the same manner as the front portion of the body with the aid of the tapered projections and mating tapered holes made in the rear portion of the body as well as the longitudinal slots and longitudinal projections on the periphery of the halves of the rear portion of the body thereby forming an overlap joint with the front portion of the body whereupon the cylindrical brace is slipped over the rear portion of the body with the double-start thread on the side of the end face until the brace is fixed on the rear cylindrical portion of the body so that the internal longitudinal guides thereof are positioned between the external longitudinal projections, with the ends of the projections smoothly curved in the opposite directions.

### Embodiment 2 of the Invention

According to this embodiment of the invention the external surface of the rear portion of the body may be smooth without the longitudinal projections having the ends thereof smoothly curved but with the internal longitudinal projections on the internal surface of the brace; the brace in this case is fixed on the rear portion of the body owing to the force provided by surface friction of the parts and brace deformation. The brace in this case should have a diameter allowing the brace to be tightly fitted on the rear portion of the body.

### Embodiment 3 of the Invention

According to this embodiment of the invention the rear end face of the body may have no the projections along the circumference to fix the brace and the brace may have no mating recesses and in this case one part is fixed relative to the other part owing to the friction force and tight fit due to brace deformation.

A list of the accompanying drawings follows below.

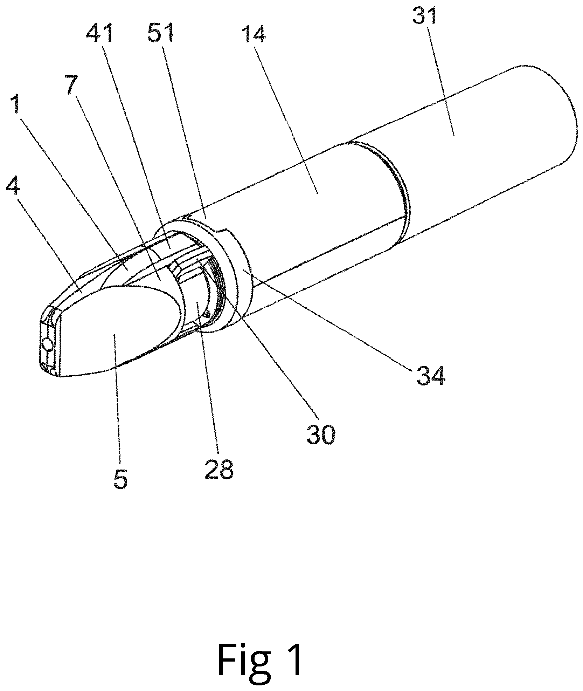

Fig. 1 is a general view of the device body, in which:
   1 - front portion of the body
   4 - truncated portion of the front portion of the body
   5 - recesses for fingers
   7 - cylindrical portion of the front portion of the body,
   14 - rear portion of the body
   28- retainer
   30- retainer lugs
   31- brace
   34- ring to fix the rear portion of the body
   41- thickened wall of the front portion of the body in the zone between the openings for the retainer lugs
   51- trapezoidal projection of the rear portion of the body to engage the ring.

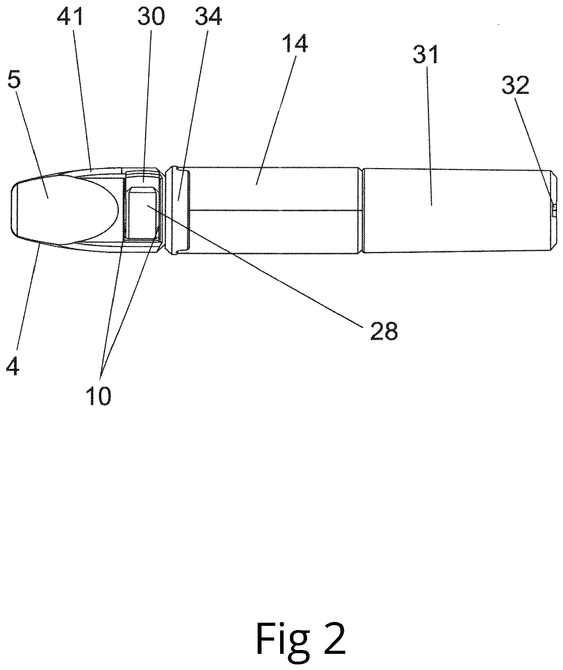

Fig. 2 is a side view of the device body, in which:
   4 - truncated portion of the front portion of the body
   5 - recesses for fingers
   10- point projection to hold the retainer lugs
   14 - rear portion of the body
   28- retainer
   30- retainer lugs
   31- brace
   32- recesses along the circumference of the brace end face for attachment to the rear portion of the body
   34- ring to fix the rear portion of the body
   41 - thickened wall of the front portion of the body in the zone between the openings for the retainer lugs.

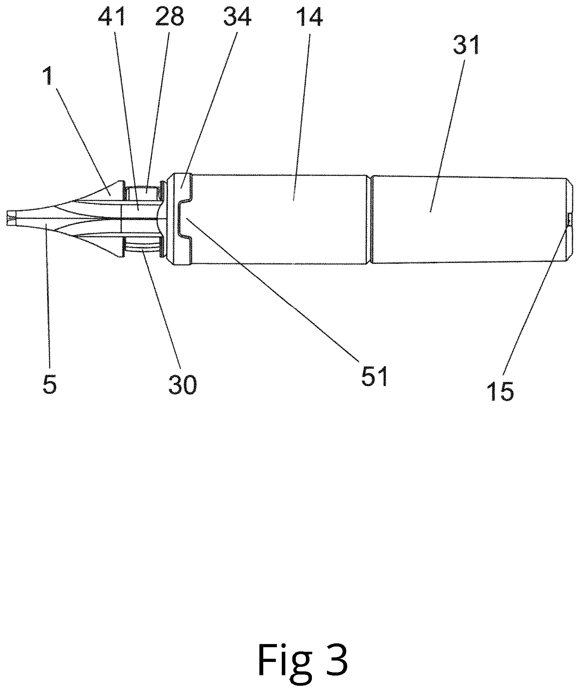

**Fig.3** is a top view of the body, in which:
   1 - front portion of the body
   5 - recesses for fingers,
   15- projections on the rear end face of the body along the circumference to fix the brace 28- retainer
   30- retainer lugs
   31- brace
   34- ring to fix the rear portion of the body
   41- thickened wall of the front portion of the body in the zone between the openings for the retainer lugs
   51- trapezoidal projection of the rear portion of the body for fixing in the ring.

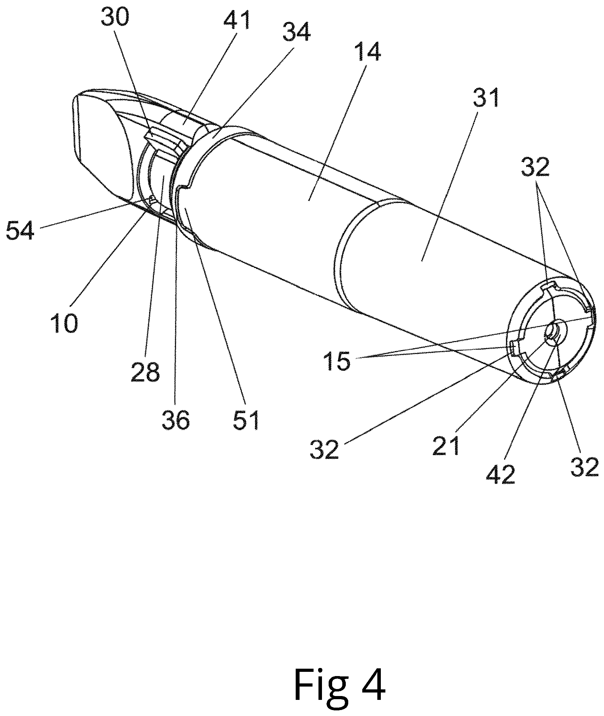

**Fig. 4**is a general view of the device body, in which:
   10- point projection to hold the retainer lugs
   14 - rear portion of the body
   15- projections on the rear end face of the body along the circumference to fix the brace
   21- through hole for the coronary stent delivery system and catheter transducer
   28- retainer
   30- retainer lugs
   31- brace
   32- recesses along the circumference of the brace end face for attachment to the rear portion of the body
   34- ring to fix the rear portion of the body
   36- trapezoidal slot of the ring to fix the rear portion of the body
   41- thickened wall of the front portion of the body in the zone between the openings for the retainer lugs
   42 - chamfer with the hole for the delivery system in the rear portion of the body
   51- trapezoidal projection of the halves of the rear portion of the body to fix by the ring
   54 - flat wall of the point projections in the square openings for the retainer lugs.

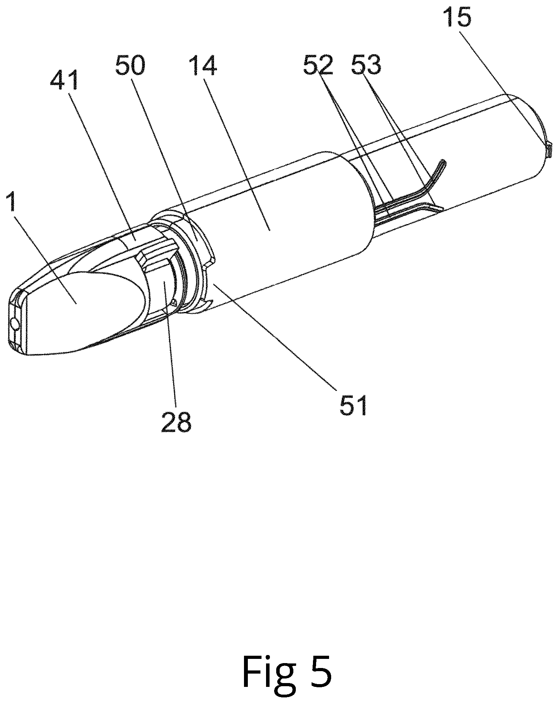

Fig. **5**is a view of the device body without the brace, in which:
   1 - front portion of the body
   14 - rear portion of the body
   15- projections on the rear end face of the body along the circumference to fix the brace
   28- retainer
   41- thickened wall of the front portion of the body in the zone between the openings for the retainer lugs
   50 -projection along the circumference of the halves of the rear portion of the body to couple by the ring
   51- trapezoidal projection of the halves of the rear portion of the body to fix by the ring
   52- longitudinal projections on the external side of the rear portion of the body to center the brace
   53- smoothly curved ends of the longitudinal projection on the external surface of the rear portion of the body.

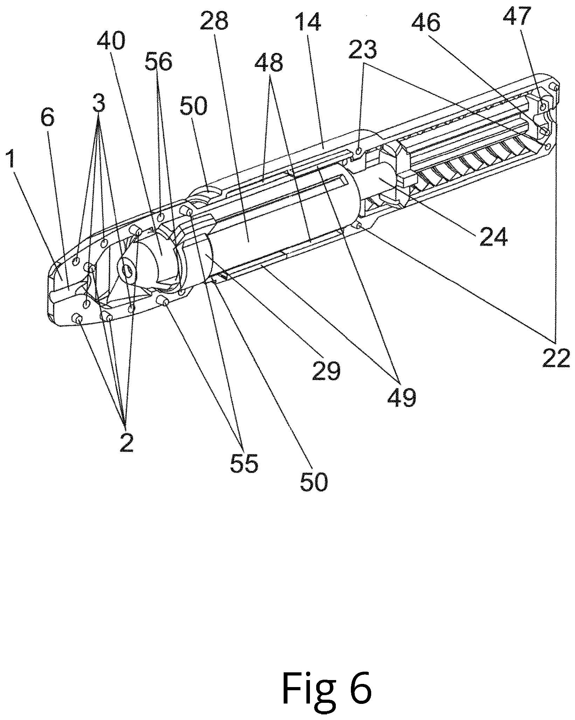

Fig. 6 is a sectional view of the body with the retainer, in which:
   1 - front portion of the body,
   2- tapered projection of the front portion of the body
   3 - tapered hole of the front portion of the body
   6 - hole for the delivery system and catheter transducer in the front portion of the body 14 - rear portion of the body
   22 - tapered projection of the rear portion of the body
   23 - tapered hole of the rear portion of the body
   24 - runner
   28- retainer
   29 - retainer thickening
   40 - cap
   46 - tapered projection of the transversal wall at the guide end
   47 - tapered hole of the transversal wall at the guide end
   48 - longitudinal slots of the internal smooth rear portion of the body
   49 - longitudinal projections of the internal smooth rear portion of the body
   50 - projection along the circumference of the halves of the rear portion of the body to couple by the ring,
   55 - tapered projection of the thickened wall of the front portion of the body
   56 - tapered hole of the thickened wall of the front portion of the body.

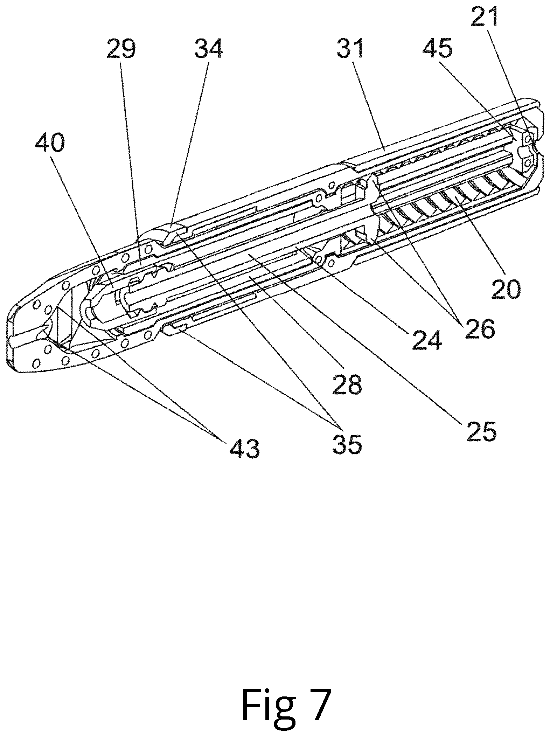

**Fig.7** is a sectional view of the body with the offset thread, in which:
   20 - internal double-start thread of the rear portion of the body for engagement with the runner
   21- through hole for the coronary stent delivery system and catheter transducer in the end face of the body
   24 - runner
   25 - through hole in the runner for the coronary stent delivery system and catheter transducer
   26 - runner projection to engage the thread of the internal surface of the rear portion of the body
   28- retainer
   29 - retainer thickening
   31- brace
   34- ring to fix the rear portion of the body
   35 - recess along the internal circumference of the ring to couple the halves of the rear portion of the body
   40- cap
   43- walls of the tapered hole of the front portion of the body rounded on the internal side
   45 - transversal wall at the end of the guide of the front portion of the body.

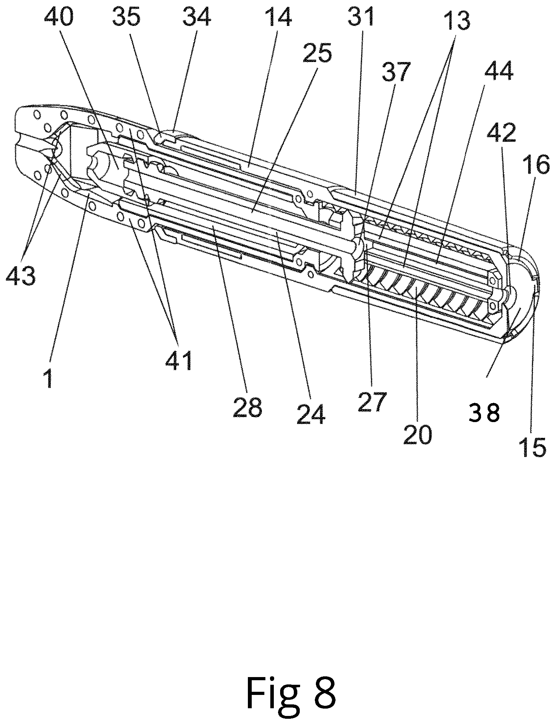

Fig. 8 is a sectional view of the body with the brace, in which:
   1 - front portion of the body,
   13- runner guides
   14 - rear portion of the body
   15 - projections to fix the brace
   16 - chamfer of the rear end face of the body
   20 - internal double-start thread of the rear portion of the body for engagement with the runner
   24 - runner
   25 - hole in the runner for the delivery system or catheter transducer
   27- projection for the guides of the front portion of the body
   28- retainer
   31 - cylindrical brace
   34- ring to fix the rear portion of the body
   35 - recess along the internal circumference of the ring to couple the halves of the rear portion of the body,
   37 - hole for the delivery system and catheter transducer in the runner
   38 -end face of the rear portion of the body
   40 - cap
   41- thickened wall of the front portion of the body in the zone between the openings for the retainer lugs
   42 -hole for the delivery system and catheter transducer in the rear portion of the body
   43 - walls of the tapered hole of the front portion of the body rounded on the internal side.

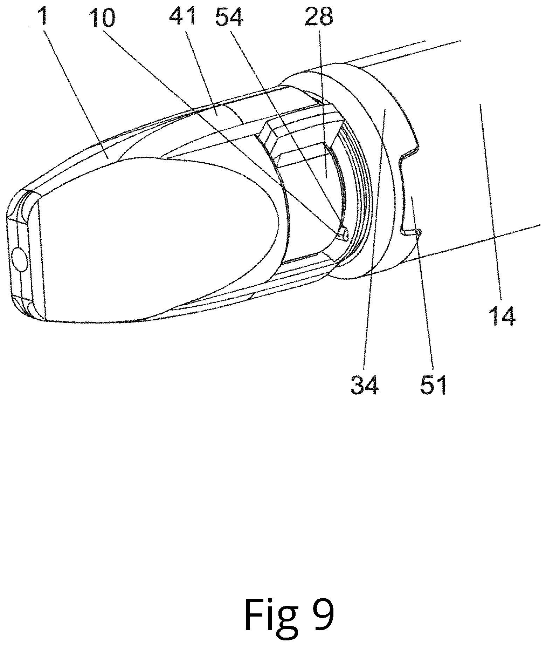

Fig. 9 is a general view of the front portion of the body coupled with the rear portion of the body, in which:
   1 - front portion of the body
   10- point projection to hold the retainer lugs
   14 - rear portion of the body
   28- retainer
   34- ring to fix the rear portion of the body
   41- thickened wall of the front portion of the body in the zone between the openings for the retainer lugs
   51- trapezoidal projection of the halves of the rear portion of the body to fix by the ring 54 - flat wall of the point projections in the square openings for the retainer lugs.

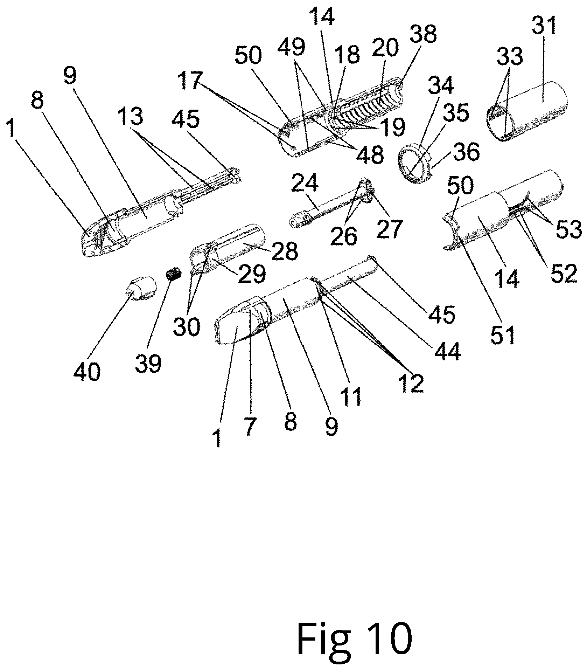

**Fig.10** is an exploded general view of the device, in which:
   1 - front portion of the body,
   7 - cylindrical portion of the front portion of the body,
   8 - openings for the retainer lugs in the front cylindrical portion of the body
   9 - smooth cylindrical portion of the front portion of the body
   11 - cylindrical slot of the smooth cylindrical portion of the front portion of the body
   12 - point elements of the cylindrical slot of the front portion of the body
   13- runner guides
   14 - rear portion of the body
   17 - point elements of the internal smooth rear portion of the body
   18 - cylindrical projection
   19 - point elements of the cylindrical projection
   20 - internal double-start thread of the rear portion of the body
   24 - runner
   26 - runner guides to engage the thread of the internal surface of the rear portion of the body
   27- projection for the guides of the front portion of the body
   28- retainer
   29 - retainer thickening
   30- retainer lugs
   31- brace
   33 - internal longitudinal projections on the brace
   34- ring to fix the rear portion of the body
   35 - recess along the internal circumference of the ring to couple the halves of the rear portion of the body
   36 - trapezoidal slot of the ring to fix the rear portion of the body
   38 - end face of the rear portion of the body
   44 - longitudinal walls to couple the guides of the front portion of the body
   45 - transverse walls at the end of the guide of the front portion of the body
   48 - longitudinal slots of the internal smooth rear portion of the body
   49 - longitudinal projections of the internal smooth rear portion of the body
   50 - projection along the circumference of the halves of the rear portion of the body to couple by the ring,
   51- trapezoidal projection of the halves of the rear portion of the body to fix by the ring
   52- longitudinal projections on the external side of the rear portion of the body to center the brace
   53- smoothly curved ends of the longitudinal projection on the external surface of the rear portion of the body.

Now the invention will be described in greater detail with reference to a specific embodiment thereof taken in conjunction with the accompanying drawings.

The body of the device is composed of two components one of which is essentially a part including afront truncated portion 4 (Fig. 1, Fig. 2) on one side thereof and a cylindrical portion 7 (Fig. 1, Fig. 10) on the opposite side thereof, a smooth cylindrical portion 9 (Fig. 10) of a smaller diameter and guides 13 (Fig. 8, Fig. 10) for a runner 24 (Fig. 6, Fig. 7, Fig. 8, Fig. 10) with a chamfer 37 (Fig. 8) with a through hole 21 (Fig. 4, Fig. 7) for a delivery system or a catheter transducer and comprises a front portion 1 (Fig. 1, Fig. 3, Fig. 5, Fig. 6, Fig. 8, Fig. 9, Fig. 10) of the body, and the other component includes a part arranged over the smooth cylindrical portion 9 (Fig. 10) of the first component and a rear part with an internal double-start thread 20 (Fig. 7, Fig. 8, Fig. 10) and a chamfer 16 (Fig. 8) on an end face 38 (Fig. 8) with the through hole 21 (Fig. 4, Fig. 7)for the delivery system or catheter transducer, said second component comprising a rear portion 14 (Fig. 1, Fig. 2, Fig. 3, Fig. 4, Fig. 5, Fig. 6, Fig. 8, Fig. 9, Fig. 10) of the body.

The front portion of the body is the front portion 4(Fig. 1, Fig. 2) which is truncated on one side and continuously extends on the opposite side in the form of the cylindrical portion7 (Fig. 1, Fig. 10), the smooth cylindrical portion 9 (Fig. 10) of a smaller diameter and the guides 13 (Fig. 8, Fig. 10) for the runner 24 (Fig. 6, Fig. 7, Fig. 8, Fig. 10) interconnected by longitudinal walls 44 (Fig. 8, Fig. 10)and provided at the end on the end face side of the body with transversal walls 45 (Fig. 7, Fig. 10) having tapered holes 46 (Fig. 6) and mating tapered projections 47 (Fig. 6) to couple the body halves.

The front truncated portion 4 (Fig. 1, Fig. 2)of the body is adapted to be held by fingers of the surgeon's left hand and provided to this end with recesses 5 (Fig. 1, Fig. 2, Fig. 3)allowing the surgeon to fix the device in the desired position, with the recesses 5 (Fig. 1, Fig. 2, Fig. 3)also serving as a pad to fix a coronary catheter. For more convenient arrangement of the coronary stent delivery system or catheter transducer, walls 43(Fig. 7, Fig. 8)in the front truncated portion 4(Fig. 1, Fig. 2)of the body are made rounded on the internal side opposite to the recesses 5 (Fig. 1, Fig. 2, Fig. 3)for the operator's left hand fingers where said walls contact the mouth of a hole 6 in which the delivery system is installed.

Square openings 8 (Fig. 10) for lugs 30 (Fig. 1, Fig. 2, Fig. 3, Fig. 4, Fig. 10) of a retainer 28 (Fig. 1, Fig. 2, Fig. 3, Fig. 4, Fig. 5, Fig. 6, Fig. 7, Fig. 8, Fig. 9, Fig. 10) are provided on both sides of the front cylindrical portion of the body 1 (Fig. 1, Fig. 3, Fig. 5, Fig. 6, Fig. 8, Fig. 9, Fig. 10) and, in the zone between said holes, walls of the front portion of the body 1 (Fig. 1, Fig. 3, Fig. 5, Fig. 6, Fig. 8, Fig. 9, Fig. 10) near the front truncated portion 4 (Fig. 1, Fig. 2) have a thickening 41 (Fig. 1, Fig. 2, Fig. 3, Fig. 4, Fig. 5, Fig. 8, Fig. 9). In each square opening 8 (Fig. 10) two point projections 10 (Fig. 2, Fig. 4, Fig. 9) are located on one side at a distance equal to the width of the lug 30 (Fig. 1, Fig. 2, Fig. 3, Fig. 4, Fig. 10) of the retainer 28 (Fig. 1, Fig. 2, Fig. 3, Fig. 4, Fig. 5, Fig. 6, Fig. 7, Fig. 8, Fig. 9, Fig. 10), said projections designed to hold the lugs 30 (Fig. 1, Fig. 2, Fig. 3, Fig. 4, Fig. 10) of the retainer 28 (Fig. 1, Fig. 2, Fig. 3, Fig. 4, Fig. 5, Fig. 6, Fig. 7, Fig. 8, Fig. 9, Fig. 10) in the extreme position and the retainer 28 (Fig. 1, Fig. 2, Fig. 3, Fig. 4, Fig. 5, Fig. 6, Fig. 7, Fig. 8, Fig. 9, Fig. 10) proper in the closed position, each projection having one flat wall 54 (Fig. 9), which is directed at an angle to a projection 10 (Fig. 2, Fig. 4, Fig. 9) on the side of the retainer open position and along which the lug 30 (Fig. 1, Fig. 2, Fig. 3, Fig. 4, Fig. 10) of the retainer 28 (Fig. 1, Fig. 2, Fig. 3, Fig. 4, Fig. 5, Fig. 6, Fig. 7, Fig. 8, Fig. 9, Fig. 10) slides when it changes its position, whereby the force to be applied by the operator's hand to rotate the lugs 30 (Fig. 1, Fig. 2, Fig. 3, Fig. 4, Fig. 10) of the retainer 28 (Fig. 1, Fig. 2, Fig. 3, Fig. 4, Fig. 5, Fig. 6, Fig. 7, Fig. 8, Fig. 9, Fig. 10) to close the mechanism is decreased.

The front smooth cylindrical portion 9 (Fig. 10) of the body has a ring 34 (Fig. 1, Fig. 2, Fig. 3, Fig. 4, Fig. 7, Fig. 8, Fig. 9, Fig. 10) suitably mounted thereon.

The ring 34 (Fig. 1, Fig. 2, Fig. 3, Fig. 4, Fig. 7, Fig. 8, Fig. 9, Fig. 10) has a recess 35 (Fig. 7, Fig. 8, Fig. 10) made along the entire internal circumference thereof and designed to receive mating projections 50 (Fig. 5, Fig. 6, Fig. 10) located along the circumference of the halves of the rear portion 14 (Fig. 1, Fig. 2, Fig. 3, Fig. 4, Fig. 5, Fig. 6, Fig. 8, Fig. 9, Fig. 10) of the body on the side of the retainer 28 (Fig. 1, Fig. 2, Fig. 3, Fig. 4, Fig. 5, Fig. 6, Fig. 7, Fig. 8, Fig. 9, Fig. 10)so as couple these halves.

On the external circumference of the ring 34 (Fig. 1, Fig. 2, Fig. 3, Fig. 4, Fig. 7, Fig. 8, Fig. 9, Fig. 10), trapezoidal slots 36 (Fig. 4, Fig. 10) are provided to receive the mating projections 51 (Fig. 1,Fig. 3, Fig. 4, Fig. 5, Fig. 9, Fig. 10) of the front end of the rear portion 14 (Fig. 1, Fig. 2, Fig. 3, Fig. 4, Fig. 5, Fig. 6, Fig. 8, Fig. 9, Fig. 10) of the body, said projections having a trapezoidal form and designed to fix the rear portion 14 (Fig. 1, Fig. 2, Fig. 3, Fig. 4, Fig. 5, Fig. 6, Fig. 8, Fig. 9, Fig. 10) of the body in the ring 34 (Fig. 1, Fig. 2, Fig. 3, Fig. 4, Fig. 7, Fig. 8, Fig. 9, Fig. 10) to prevent its spontaneous rotation.

A cylindrical slot 11 (Fig. 10) with point elements 12 (Fig. 10) is provided along the circumference of the end face of the smooth cylindrical portion of the front portion 9 (Fig. 10) of the body on the side of the guide 13 (Fig. 8, Fig. 10) of the runner 24 (Fig. 6, Fig. 7, Fig. 8, Fig. 10), said cylindrical slot designed for coupling with a mating projection 18 (Fig. 10) with point elements 19 (Fig. 10) made along the circumference of the internal smooth cylindrical surface of the rear portion 14 (Fig. 1, Fig. 2, Fig. 3, Fig. 4, Fig. 5, Fig. 6, Fig. 8, Fig. 9, Fig. 10) of the body on the side of the surface with the double-start thread 20 (Fig. 7, Fig. 8, Fig. 10).

The rear cylindrical portion 14 (Fig. 1, Fig. 2, Fig. 3, Fig. 4, Fig. 5, Fig. 6, Fig. 8, Fig. 9, Fig. 10) of the body has longitudinal projections 49 (Fig. 6, Fig. 9) and mating longitudinal slots 48 (Fig. 6, Fig. 9) located on the internal side thereof along the edges of the halves in the smooth cylindrical surface zone and used to couple the halves of the rear portion 14 (Fig. 1, Fig. 2, Fig. 3, Fig. 4, Fig. 5, Fig. 6, Fig. 8, Fig. 9, Fig. 10) of the body.

Point elements 17 (Fig. 10)arranged on the internal smooth cylindrical surface of the rear portion 14 (Fig. 1, Fig. 2, Fig. 3, Fig. 4, Fig. 5, Fig. 6, Fig. 8, Fig. 9, Fig. 10) of the body along the circumference on the retainer side are designed to reduce friction between the rotatable portions of the body.

The cylindrical projection 18 (Fig. 10) with the point elements 19 (Fig. 10) is provided along the circumference between the smooth internal surface and the surface with the internal double-start thread 20 (Fig. 7, Fig. 8, Fig. 10) of the rear portion 14 (Fig. 1, Fig. 2, Fig. 3, Fig. 4, Fig. 5, Fig. 6, Fig. 8, Fig. 9, Fig. 10) of the body to engage the cylindrical slot 11 (Fig. 10) of the smooth cylindrical part of the front portion 9 (Fig. 10) of the body on the side of the guide 13 (Fig. 8, Fig. 10) of the runner 24 (Fig. 6, Fig. 7, Fig. 8, Fig. 10) so that the rear cylindrical portion 14 (Fig. 1, Fig. 2, Fig. 3, Fig. 4, Fig. 5, Fig. 6, Fig. 8, Fig. 9, Fig. 10) of the body can be rotated about the axis thereof.

Longitudinal projections 52 (Fig. 5, Fig. 10) are made on the external surface of the rear portion 14 (Fig. 1, Fig. 2, Fig. 3, Fig. 4, Fig. 5, Fig. 6, Fig. 8, Fig. 9, Fig. 10) of the body, with ends 53 (Fig. 5, Fig. 10) smoothly curved in the opposite directions in order to center a brace 31(Fig. 1, Fig. 2, Fig. 3, Fig. 4, Fig. 7, Fig. 8, Fig. 10) fitted over the rear portion 14 (Fig. 1, Fig. 2, Fig. 3, Fig. 4, Fig. 5, Fig. 6, Fig. 8, Fig. 9, Fig. 10) of the body and to prevent spontaneous rotation of the brace. Projections 15 (Fig. 3, Fig. 4, Fig. 5, Fig. 8) to fix the brace 31(Fig. 1, Fig. 2, Fig. 3, Fig. 4, Fig. 7, Fig. 8, Fig. 10) in recesses 32 (Fig. 3, Fig. 4) along the circumference of the end face of the brace 31(Fig. 1, Fig. 2, Fig. 3, Fig. 4, Fig. 7, Fig. 8, Fig. 10) are located on the end face 38 (Fig. 8) of the rear portion 14 (Fig. 1, Fig. 2, Fig. 3, Fig. 4, Fig. 5, Fig. 6, Fig. 8, Fig. 9, Fig. 10) of the body.

The brace 31 (Fig. 1, Fig. 2, Fig. 3, Fig. 4, Fig. 7, Fig. 8, Fig. 10) is fitted over the rear portion 14 (Fig. 1, Fig. 2, Fig. 3, Fig. 4, Fig. 5, Fig. 6, Fig. 8, Fig. 9, Fig. 10) of the body in the zone of the internal double-start thread 20 (Fig. 7, Fig. 8, Fig. 10).

The brace 31 (Fig. 1, Fig. 2, Fig. 3, Fig. 4, Fig. 7, Fig. 8, Fig. 10) is essentially a hollow cylinder with internal longitudinal guides 33 along the whole length to ensure snug fit to the external surface of the rear portion 14 (Fig. 1, Fig. 2, Fig. 3, Fig. 4, Fig. 5, Fig. 6, Fig. 8, Fig. 9, Fig. 10) of the body on the external side of which the mating longitudinal projections 52 (Fig. 5, Fig. 10) are provided with the ends 53 (Fig. 5, Fig. 10) thereof smoothly curved in the opposite directions, said projections used to securely attach the brace (Fig. 1, Fig. 2, Fig. 3, Fig. 4, Fig. 7, Fig. 8, Fig. 10).

The recesses 32 (Fig. 2, Fig. 4) made along the circumference of the end face of the brace 31 (Fig. 1, Fig. 2, Fig. 3, Fig. 4, Fig. 7, Fig. 8, Fig. 10) serve to fix the brace 31 (Fig. 1, Fig. 2, Fig. 3, Fig. 4, Fig. 7, Fig. 8, Fig. 10) on the surface of the rear portion 14 (Fig. 1, Fig. 2, Fig. 3, Fig. 4, Fig. 5, Fig. 6, Fig. 8, Fig. 9, Fig. 10) of the body with the aid of mating projections 15 (Fig. 3, Fig. 4, Fig. 5, Fig. 8) along the circumference of the end face 38 (Fig. 8) of the rear portion 14 (Fig. 1, Fig. 2, Fig. 3, Fig. 4, Fig. 5, Fig. 6, Fig. 8, Fig. 9, Fig. 10) of the body.

Through holes 6 (Fig. 6), 21 (Fig. 4, Fig. 7) and 25 (Fig. 7, Fig. 8) are made in the front portion 1 (Fig. 1, Fig. 3, Fig. 5, Fig. 6, Fig. 8, Fig. 9, Fig. 10) of the body, the end face 38 of the rear portion 14 (Fig. 1, Fig. 2, Fig. 3, Fig. 4, Fig. 5, Fig. 6, Fig. 8, Fig. 9, Fig. 10) of the body, the runner 24 (Fig. 6, Fig. 7, Fig. 8, Fig. 10), a cap 40 (Fig. 6, Fig. 7, Fig. 8, Fig. 10) and an elastic bushing 39 (Fig. 10)to introduce the coronary stent delivery system or pull catheter transducer into the device installed in the body.

For easy positioning of the coronary stent on the delivery system or catheter transducer inside the device in the central through hole 6 (Fig. 6), the central hole 21 (Fig. 4, Fig. 7) and a central hole 25 (Fig. 7, Fig. 8), a chamfer 42 (Fig. 8) with the hole 21 (Fig. 7) is made on the end face 38 (Fig. 8) of the rear portion 14 (Fig. 1, Fig. 2, Fig. 3, Fig. 4, Fig. 5, Fig. 6, Fig. 8, Fig. 9, Fig. 10) of the body whereas the chamfer 37 (Fig. 8) with the hole 25 (Fig. 7) is made on the end face of the runner 24 (Fig. 6, Fig. 7 Fig. 8, Fig. 10).

The body comprises the front portion 1 (Fig. 1, Fig. 3, Fig. 5, Fig. 6, Fig. 8, Fig. 9, Fig. 10) and the rear portion 14 (Fig. 1, Fig. 2, Fig. 3, Fig. 4, Fig. 5, Fig. 6, Fig. 8, Fig. 9, Fig. 10) made of pressure cast plastic and is composed of two identical halves which are coupled after the mechanism is assembled.

The front portion of the body is assembled with the aid of tapered projections 2 (Fig. 6), 55 (Fig. 6) and mating tapered holes 3 (Fig. 6), 56 (Fig. 6) made along the internal edges of the front portion 1 (Fig. 1, Fig. 3, Fig. 5, Fig. 6, Fig. 8, Fig. 9, Fig. 10) of the body, with the maximum possible number of the tapered projections 3 (Fig. 6) and the mating tapered holes 2 (Fig. 6) made on the tapered projection 22 (Fig. 6) of the front portion 1 (Fig. 1, Fig. 3, Fig. 5, Fig. 6, Fig. 8, Fig. 9, Fig. 10) of the body, tapered projections 55 (Fig. 6) and the mating tapered holes 56 (Fig. 6) in the zone between the lugs 30 (Fig. 1, Fig. 2, Fig. 3, Fig. 4, Fig. 10) of the retainer 28 (Fig. 1, Fig. 2, Fig. 3, Fig. 4, Fig. 5, Fig. 6, Fig. 7, Fig. 8, Fig. 9, Fig. 10) and the tapered projections 46 (Fig 6) with the mating tapered holes 47 (Fig. 6) on the transversal walls (Fig. 7, Fig. 10) at the end of the guides 13 (Fig. 8) of the runner 24 (Fig. 6, Fig. 7, Fig. 8, Fig. 10) of the front portion 1 (Fig. 6) of the body, whereby jointing reliability is made higher.

After the halves of the front portion 1 (Fig. 1, Fig. 3, Fig. 5, Fig. 6, Fig. 8, Fig. 9, Fig. 10) of the body are coupled, the ring 34 (Fig. 1, Fig. 2, Fig. 3, Fig. 4, Fig. 7, Fig. 8, Fig. 9, Fig. 10) is fitted over the smooth cylindrical portion 9 (Fig. 10) of the front portion of the body on the side of the guides 13 (Fig. 8) for the runner 24 (Fig. 6, Fig. 7, Fig. 8, Fig. 10), said ring having the recess 35 (Fig. 7, Fig. 8, Fig. 10) in which the mating projections 50 (Fig. 6, Fig. 10) of the semicircles of the halves of the rear portion 14 (Fig. 1, Fig. 2, Fig. 3, Fig. 4, Fig. 5, Fig. 6, Fig. 8, Fig. 9, Fig. 10) of the body are inserted whereas in the trapezoidal slots 36 (Fig. 10) made on the external circumference of the ring 34 (Fig. 2,Fig. 3, Fig. 4, Fig. 7, Fig. 8, Fig. 9, Fig. 10) the mating trapezoidal projections 51 (Fig. 1, Fig. 3, Fig. 4, Fig. 5, Fig. 9, Fig. 10) on the semicircles of the halves of the front portion of the rear portion 14 (Fig. 1,Fig. 2, Fig. 3, Fig. 4, Fig. 5, Fig. 6, Fig. 8, Fig. 9, Fig. 10) of the body are inserted whereupon the halves are coupled so that the smooth cylindrical portion of the front portion 9 (Fig. 10) of the body is accommodated in the halves of the rear smooth cylindrical portion 14 (Fig. 1, Fig. 2, Fig. 3, Fig. 4, Fig. 5, Fig. 6, Fig. 8, Fig. 9, Fig. 10) of the body, wherein the cylindrical projection 18 (Fig. 10) and the point elements 19 (Fig. 10)located between the internal smooth portion of the rear portion 14 (Fig. 10) of the body and the portion having the internal double-start thread 20 (Fig. 10) are inserted in the cylindrical slot 11 (Fig. 10) with the point elements 12 (Fig. 10) of the smooth cylindrical portion of the front portion 9 (Fig. 10) of the body on the side of the runner guides and the halves are coupled in the same manner as the front portion of the body with the aid of the tapered projections 22 (Fig. 6) and mating tapered holes 23 (Fig. 6) made in the rear portion 14 (Fig. 1, Fig. 2, Fig. 3, Fig. 4, Fig. 5, Fig. 6, Fig. 8, Fig. 9, Fig. 10) of the body and with the aid of the longitudinal slots 48 (Fig. 6, Fig. 10) and the longitudinal projections 49 (Fig. 6, Fig. 10) along the edges of the halves of the rear internal smooth part of the rear portion of the body thereby forming an overlap joint with the front portion 1 (Fig. 1, Fig. 3, Fig. 5, Fig. 6, Fig. 8, Fig. 9, Fig. 10) of the body whereupon the cylindrical brace 31 (Fig. 1, Fig. 2, Fig. 3, Fig. 4, Fig. 7, Fig. 8, Fig. 10) is slipped over the rear portion 14 (Fig. 1, Fig. 2, Fig. 3, Fig. 4, Fig. 5, Fig. 6, Fig. 8, Fig. 9, Fig. 10) of the body in the zone of the internal double-start thread 20 (Fig. 7, Fig. 8, Fig. 10) on the side of the end face 38 (Fig. 8) until said brace is fixed on the rear cylindrical portion 14 (Fig. 1, Fig. 2, Fig. 3, Fig. 4, Fig. 5, Fig. 6, Fig. 8, Fig. 9, Fig. 10) of the body so that the internal longitudinal guides 33 (Fig. 10) thereof are positioned between the external longitudinal projections 52 (Fig. 5, Fig. 10) of the rear portion of the body, with the ends 53 (Fig. 5, Fig. 10) of said projections smoothly curved in the opposite directions.

### Embodiment 2 of the Invention

According to this embodiment of the invention the external surface of the rear portion 14 (Fig. 1, Fig. 2, Fig. 3, Fig. 4, Fig. 5, Fig. 6, Fig. 8, Fig. 9, Fig. 10) of the body may be smooth without the external longitudinal projections 52 (Fig. 5, Fig. 10) having the ends 53 (Fig. 5, Fig. 10) thereof smoothly curved but with the internal longitudinal projections 33 (Fig. 10) on the internal surface of the brace 31 (Fig. 10); the brace 31 (Fig. 1, Fig. 2, Fig. 3, Fig. 4, Fig. 7, Fig. 8, Fig. 10) in this case is fixed on the rear portion 14 (Fig. 1, Fig. 2, Fig. 3, Fig. 4, Fig. 5, Fig. 6, Fig. 8, Fig. 9, Fig. 10) of the body owing to the force provided by surface friction of the parts and brace deformation. The brace 31 (Fig. 1, Fig. 2, Fig. 3, Fig. 4, Fig. 7, Fig. 8, Fig. 10) in this case should have a diameter allowing the brace to be tightly fitted on the rear portion 14 (Fig. 1, Fig. 2, Fig. 3, Fig. 4, Fig. 7, Fig. 8, Fig. 9, Fig. 10) of the body.

### Embodiment 3 of the Invention

According to this embodiment of the invention the rear end face 38 (Fig. 8) of the body may have no projections 15 (Fig. 3, Fig. 8) along the circumference to fix the brace 31 (Fig. 1, Fig. 2, Fig. 3, Fig. 4, Fig. 7, Fig. 8, Fig. 10) and the brace 31 (Fig. 1, Fig. 2, Fig. 3, Fig. 4, Fig. 7, Fig. 8, Fig. 10) may have no mating holes 32 (Fig. 2, Fig. 4) to receive said projections 15 (Fig. 3, Fig. 8) and in this case one part is fixed relative to the other part owing to the friction force and tight fit provided by deformation of the brace 31 (Fig. 1, Fig. 2, Fig. 3, Fig. 4, Fig. 7, Fig. 8, Fig. 10).

## Claims

1. A body of a device suitable for positioning a coronary stent in coronary arteries or pulling a catheter transducer for intravascular ultrasound, said body comprising a retainer (28) with lugs (30), a runner (24) with an opening (25) to accommodate a delivery system therein and a chamfered (16) rear portion (14), said runner (24) provided with projections (26) to engage an internal thread (20) of a rear cylindrical portion (14) of the body and projections (27) for movement along guides (13) inside the body on one side and with a cap (40) with a hole having an elastic bushing (39) with an internal hole with tapered end surfaces conforming to surfaces inside the cap (40) and the runner (24), said bushing (39) positioned in the threaded cap (40) to be screwed on the runner (24) on the opposite side,
wherein the body is composed of the front (1) and rear (14) portions made of two halves;
the front portion (1) of the body is truncated (4) on one side thereof and further extends on the opposite side thereof in the form of a cylindrical portion (7) with openings (8) for retainer (28) lugs (30) with point projections (10) in each opening (8), and extends in the form of a smooth cylindrical portion (9) of a smaller diameter and runner (24) guides (13) with a cylindrical slot (11) with point elements (12) in front of the guides (13), whereas the rear portion (14) of the body is made cylindrical and has an internal smooth surface, having point elements (17) along the circumference thereof, and a surface with a double-start thread (20) along the circumference thereof, wherein a cylindrical projection (18) with a point element (19) is provided between the smooth internal surface and the surface with the double-start thread (20) along the circumference thereof, the end face (38) of the rear portion (14) has a chamfer (16) and a through hole (6, 21) is made in the front portion (1) and the end face (38) of the rear portion (14) to introduce the coronary stent delivery system or the catheter transducer for intravascular ultrasound into the device interior,
the front portion (1) of the body comprising:
- rounded walls (43) in the truncated front portion (4) of the body on the internal side opposite to recesses (5) for the fingers of the operator's left hand in a place where the said walls (43) adjoin the mouth of the input hole (6) to accommodate the delivery system or the catheter transducer,
- thickened walls **(41)** in the zone between the opening (8) for the retainer (28) lugs (30) of the front (1) portion of the body,
- flat walls (54) located at an angle to the point projections (10) in the openings (8) the for the retainer (28) lugs (30),
**characterized by**:
- a ring (34) rotatably mounted on the smooth cylindrical surface (9) of the front portion (1) of the body, the ring (34) having a recess (35) in which projections (50) of halves of the rear portion (14) of the body are located along the circumference to couple said halves of the rear portion (14) of the body,
- trapezoidal slots (36) trapezoidal projections (51) of the halves of the rear portion (14) of the body are located along the circumference to couple said halves of the rear portion (14) of the body,
- longitudinal walls (44) connecting the runner (24) guides (13) and transversal walls (45) adjoining at the end of the guides (13) are provided with a tapered projection (47) and a mating tapered hole (46) and the chamfered (37) runner (24) near a central hole (25),
the rear portion (14) of the body comprising:
- longitudinal projections (49) and mating slots (48) along the edges of the internal smooth portion of the halves of the body to couple said halves of the body,
- longitudinal projections (52) having the ends (53) thereof curved in the opposite directions on the external surface of the body in the zone with the internal double-start thread (20) to engage the longitudinal projections (33) along the circumference of the internal surface of a brace (31),
the brace (31) having the form of a hollow cylinder and provided with:
- longitudinal projections (33) along the circumference of the internal surface to engage the longitudinal projections (52) of the rear portion (14) of the body,
- recesses (32) along the circumference of the end face of the rear portion (14) of the body for attachment in the mating projections (15), provided along the circumference of the brace (31) to hold the brace (31).

2. The body of the device as claimed in Claim 1 **characterized in that** the external surface of the rear portion (14) of said body is smooth and has no longitudinal projections (52) smoothly curved in the opposite directions on the external surface.

3. The body of the device as claimed in Claim 1 **characterized in that** the end face of the cylindrical brace (31) has no recesses (32) along the circumference thereof whereas the end face of the rear portion (14) of the body has no mating projections (15) along the circumference thereof.

## Patentansprüche

1. Ein Körper der Vorrichtung, der zum Positionieren eines Koronarstents in Koronararterien oder zum Ziehen eines Katheterwandlers für intravaskulären Ultraschall geeignet ist, wobei der Körper eine Halterung (28) mit Laschen (30), einen Läufer (24) mit einer Öffnung (25) zur Aufnahme eines Zuführungssystems darin und einen abgeschrägten (16) hinteren Abschnitt (14), wobei der Läufer (24) mit Vorsprüngen (26) zum Eingriff in ein Innengewinde (20) eines hinteren zylindrischen Abschnitts (14) des Körpers und mit Vorsprüngen (27) für die Bewegung entlang Führungen (13) innerhalb des Körpers auf einer Seite versehen ist, und mit einer Kappe (40) mit einem Loch mit einer elastischen Buchse (39) mit einem Innenloch mit konischen Endflächen, die den Flächen im Inneren der Kappe (40) und des Läufers (24) entsprechen, wobei die Buchse (39) in der Gewindekappe (40) positioniert ist, um auf den Läufer (24) auf der gegenüberliegenden Seite geschraubt zu werden, wobei der Körper aus dem vorderen (1) und dem hinteren (14) Teil besteht, die aus zwei Hälften bestehen; der vordere Teil (1) des Körpers auf einer Seite abgeschnitten (4) ist und sich auf der gegenüberliegenden Seite in Form eines zylindrischen Teils (7) mit Öffnungen (8) für Halterungslaschen (30) mit Punktvorsprüngen (10) in jeder Öffnung (8) kontinuierlich fortsetzt, ein glatter zylindrischer Abschnitt (9) mit kleinerem Durchmesser und Führungen (13) für den Lauf (24) mit einem zylindrischen Schlitz (11) mit Spitzenelementen (12) vor den Führungen (13), während der hintere Abschnitt (14) des Körpers zylindrisch ausgebildet ist und eine glatte Innenfläche mit Spitzenelementen (17) entlang seines Umfangs aufweist, und eine Oberfläche mit einem Doppelgewinde (20) entlang ihres Umfangs aufweist, wobei zwischen der glatten Innenfläche und der Oberfläche mit dem Doppelgewinde (20) entlang ihres Umfangs ein zylindrischer Vorsprung (18) mit einem Spitzenelement (19) vorgesehen ist, die Stirnfläche (38) oder der hintere Abschnitt (14) eine Abschrägung (16) und eine Durchgangsbohrung (6, 21) ausgebildet, um das Koronarstent-Zuführungssystem oder den Katheterwandler für intravaskulären Ultraschall in das Innere der Vorrichtung einzuführen, wobei
der vordere Abschnitt (1) des Körpers umfasst:
abgerundete Wände (43) im abgeschnittenen vorderen Abschnitt (4) des Körpers auf der Innenseite gegenüber den Aussparungen (5) für die Finger der linken Hand des Bedieners an einer Stelle, an der die Wände (43) an die Öffnung des Eingangslochs (6) angrenzen, um das Zuführungssystem oder den Katheterwandler aufzunehmen,
verdickte Wände (41) in dem Bereich zwischen der Öffnung (8) für die Halterungslaschen (30) des Halterungselements (28) des vorderen Abschnitts (1) des Körpers,
flache Wände (54), die in einem Winkel zu den Punktvorsprüngen (10) in den Öffnungen (8) für die Halterungslaschen (30) des Halterungselements (28) angeordnet sind,
**gekennzeichnet durch**
einen Ring (34), der drehbar auf der glatten zylindrischen Oberfläche (9) des vorderen Abschnitts (1) des Körpers angebracht ist und eine Aussparung (35) aufweist, in der Vorsprünge (50) der Hälften des hinteren Abschnitts (14) des Körpers entlang des Umfangs angeordnet sind, um die Hälften des hinteren Abschnitts (14) des Körpers zu koppeln, während in trapezförmigen Schlitzen (36) trapezförmige Vorsprünge (51) der Hälften des hinteren Abschnitts (14) des Körpers angeordnet sind, um die Hälften des hinteren Abschnitts (14) des Körpers zu fixieren, wobei
Längswände (44), die die Führungen (24) (13) und die an das Ende der Führungen (13) angrenzenden Querwände (45) sind mit einem konischen Vorsprung (47) und einer passenden konischen Bohrung (46) versehen, und der abgeschrägte (37) Läufer (24) in der Nähe einer zentralen Bohrung (25),
wobei der hintere Teil des Körpers umfasst:
Längsvorsprünge (49) und passende Schlitze (48) entlang den Kanten des inneren glatten Abschnitts der Körperhälften, um die Körperhälften zu verbinden,
Längsvorsprünge (52), deren Enden (53) in entgegengesetzte Richtungen gekrümmt sind, um mit den Längsvorsprüngen (33) entlang des Umfangs der Innenfläche einer Strebe (31) in Eingriff zu kommen, wobei
die Strebe (31) die Form eines Hohlzylinders hat und mit
Längsvorsprüngen (33) entlang des Umfangs der Innenfläche, um mit den Längsvorsprüngen (52) in Eingriff zu kommen, deren Enden (53) in entgegengesetzte Richtungen gebogen sind und sich auf der Außenfläche im Bereich des hinteren Abschnitts (14) des Körpers mit dem inneren Doppelgewinde (20) befinden,
Löchern (32) in der Stirnfläche zur Befestigung an der Stirnfläche (38) des hinteren Abschnitts (14) des Körpers zur Befestigung in den Vorsprüngen (15) entlang des Umfangs der Stirnfläche (38) des hinteren Abschnitts (14) des Körpers, um die Strebe (31) zu halten.

2. Der Körper der Vorrichtung nach Anspruch 1, **dadurch gekennzeichnet, dass** die Außenfläche des hinteren Abschnitts (14) des Körpers glatt ist und keine Längsvorsprünge (52) aufweist, die in entgegengesetzte Richtungen auf der Außenfläche glatt gekrümmt sind.

3. Der Körper der Vorrichtung nach Anspruch 1, **dadurch gekennzeichnet, dass** die Stirnfläche der zylindrischen Stütze (31) keine Löcher (32) entlang ihres Umfangs aufweist, während die Stirnfläche (38) des hinteren Abschnitts (14) des Körpers keine passenden Vorsprünge (15) entlang ihres Umfangs aufweist.

## Revendications

1. Corps de dispositif adapté pour positionner un stent coronaire dans des artères coronaires ou tirer un transducteur de cathéter pour une échographie intravasculaire, ledit corps comprenant un dispositif de retenue (28) avec des pattes **(30),** un coulisseau **(24)** avec une ouverture **(25)** pour loger un système de délivrance à l'intérieur et une partie arrière chanfreinée **(16) (14),** ledit coulisseau **(24)** étant muni de saillies **(26)** pour s'engager dans un filetage interne **(20)** d'une partie cylindrique arrière **(14)** du corps et de saillies **(27)** pour se déplacer le long de guides **(13)** à l'intérieur du corps d'un côté, et avec un capuchon **(40)** avec un trou comportant une douille élastique **(39)** avec un trou interne avec des surfaces d'extrémité coniques se conformant aux surfaces à l'intérieur du capuchon **(40)** et du coulisseau **(24),** ladite douille **(39)** étant positionnée dans le capuchon fileté **(40)** pour être vissée sur le coulisseau **(24)** sur le côté opposé, dans lequel le corps est composé des parties avant **(1)** et arrière **(14)** constituées de deux moitiés ; la partie avant **(1)** du corps est tronquée **(4)** sur un côté et s'étend de manière continue sur le côté opposé sous la forme d'une partie cylindrique **(7)** avec des ouvertures **(8)** pour des ergots de retenue **(28) (30)** avec des saillies pointues **(10)** dans chaque ouverture **(8),** partie cylindrique lisse **(9)** de plus petit diamètre et guide de glissière **(24) (13)** avec une fente cylindrique **(11)** avec des éléments en pointe **(12)** devant les guides **(13),** tandis que la partie arrière **(14)** du corps est cylindrique et présente une surface interne lisse, avec des éléments en pointe (17) le long de sa circonférence, et une surface avec un filetage à double entrée **(20)** le long de sa circonférence, dans lequel une saillie cylindrique **(18)** avec un élément en pointe **(19)** est prévue entre la surface interne lisse et la surface avec le filetage à double entrée **(20)** le long de sa circonférence, la face d'extrémité **(38)** ou la partie arrière **(14)** comporte un chanfrein **(16)** et un trou traversant **(6, 21)** est réalisé dans la partie avant **(1)** et la face d'extrémité **(38)** de la partie arrière **(14)** pour introduire le système de mise en place de stent coronaire ou le transducteur de cathéter pour ultrasons intravasculaires à l'intérieur du dispositif,
la partie avant **(1)** du corps comprenant :
des parois arrondies **(43)** dans la partie avant tronquée **(4)** du corps sur le côté interne opposé aux évidements **(5)** pour les doigts de la main gauche de l'opérateur à un endroit où lesdites parois **(43)** sont adjacentes à l'embouchure du trou d'entrée **(6)** pour accueillir le système de mise en place ou le transducteur de cathéter,
des parois épaissies **(41)** dans la zone entre l'ouverture **(8)** pour les ergots **(30)** du dispositif de retenue **(28)** de la partie avant **(1)** du corps,
des parois plates **(54)** situées à un angle par rapport aux saillies ponctuelles **(10)** dans les ouvertures **(8)** pour les ergots **(30)** du dispositif de retenue **(28),**
**caractérisé par**
une bague **(34)** montée de manière rotative sur la surface cylindrique lisse **(9)** de la partie avant **(1)** du corps, comportant un évidement **(35)** dans lequel des saillies **(50)** des moitiés de la partie arrière **(14)** du corps sont situées le long de la circonférence pour coupler lesdites moitiés de la partie arrière **(14)** du corps, tandis que dans des fentes trapézoïdales
**(36),** des saillies trapézoïdales **(51)** des moitiés de la partie arrière **(14)** du corps sont situées pour fixer lesdites moitiés de la partie arrière **(14)** du corps, des parois longitudinales **(44)** reliant les guides de glissière **(24)**
**(13)** et les parois transversales **(45)** adjacentes à l'extrémité des guides **(13)** sont pourvues d'une saillie conique **(47)** et d'un trou conique correspondant **(46),** et le coulisseau **(24)** chanfreiné **(37)** près d'un trou central **(25),**
la partie arrière du corps comprenant :
des saillies longitudinales **(49)** et des fentes correspondantes **(48)** le long des bords de la partie lisse interne des moitiés du corps pour coupler lesdites moitiés du corps,
des saillies longitudinales **(52)** dont les extrémités **(53)** courbées dans des directions opposées sur la surface externe du corps dans la zone comportant le filetage interne à double pas **(20)** pour s'engager avec les saillies longitudinales **(33)** le long de la circonférence de la surface interne d'un renfort **(31),**
le renfort **(31)** ayant la forme d'un cylindre creux et étant muni de :
des saillies longitudinales **(33)** le long de la circonférence de la surface interne pour s'engager avec les saillies longitudinales **(52)** ayant les extrémités **(53)** courbées dans des directions opposées et situées sur la surface externe dans la zone de la partie arrière **(14)** du corps avec le filetage interne à double pas **(20),**
des trous **(32)** dans la face d'extrémité pour la fixation à la face d'extrémité **(38)** de la partie arrière **(14)** du corps pour la fixation dans les saillies **(15)** le long de la circonférence de la face d'extrémité **(38)** de la partie arrière **(14)** du corps afin de maintenir l'entretoise **(31).**

2. Le corps du dispositif selon la revendication 1, **caractérisé en ce que** la surface externe de la partie arrière **(14)** dudit corps est lisse et ne comporte aucune saillie longitudinale **(52)** incurvée de manière régulière dans les directions opposées sur la surface externe.

3. Le corps du dispositif selon la revendication 1, **caractérisé en ce que** la face d'extrémité de l'entretoise cylindrique **(31)** ne comporte aucun trou **(32)** le long de sa circonférence, tandis que la face d'extrémité **(38)** de la partie arrière **(14)** du corps ne comporte aucune saillie d'accouplement **(15)** le long de sa circonférence.
